# FASCICULE DE BREVET EUROPEEN

(11) **EP 0 718 288 B1**
(45) Date de publication et mention de la délivrance du brevet: **29.06.2005**
(21) Numéro de dépôt: 95119462.0
(22) Date de dépôt: 11.12.1995
(51) Int. Cl.: C07D 233/06, H01G 9/20

(54) **Sels liquides hydrophobes, leur préparation et leur application en électrochimie**
Flüssige, hydrophobe Salze, ihre Herstellung und ihre Verwendung in der Elektrochemie
Liquid hydrophobic salts, their preparation and their use in electrochemistry

(30) Priorité: 21.12.1994 CH 386294; 27.12.1994 FR 9415704
(43) Date de publication de la demande: 26.06.1996
(73) Titulaire: HYDRO QUEBEC, Montréal H2Z 1A4, Québec (CA); CENTRE NATIONAL DE LA RECHERCHE SCIENTIFIQUE (CNRS), 75794 Paris Cedex 16 (FR)
(72) Inventeur: Bonhôte, Pierre, CH-2000 Neuchâtel (CH); Dias, Ana-Paula, CH-2006 Lausanne (CH)
(74) Mandataire: Guerre, Dominique

(56) Documents cités:
- EP-A- 0 096 629
- PROC. - ELECTROCHEM. SOC., vol. 16, 1992 pages 386-396, E.I. COOPER ET AL. 'New, stable ambient-temperature molten salts'

## Description

La présente invention a pour objet de nouveaux sels hydrophobes, liquides et de faible viscosité à température ambiante, ayant pour cation un N,N'-dialkylimidazolium et pour anion le bis-trifluorométhanesulfonylamidure (CF₃SO₂)₂N⁻(autrement dénommé bis-triflylamidure), ainsi que leur procédé de fabrication.

L'invention concerne également l'application desdits sels en tant que solvants polaires hydrophobes pour des applications électrochimiques ou synthétiques, par exemple en tant que solvant dans une composition électrolytique pour cellules photovoltaïques électrochimiques.

On connaît déjà des sels liquides à température ambiante ayant pour cation et pour anion respectivement un imidazolium substitué et le trifluorométhane sulfonate (autrement dénommé triflate). De tels composés sont décrits dans une publication de E. J. Cooper et E. J. M. O'Sullivan (The Electrochem. Soc., Proceeding Vol. 92-16 (1992)). Ces composés ont des viscosités variées, mais tous sont miscibles à l'eau (hydrophiles) à l'exception du triflate de dodécyl-1-éthyl-3 imidazolium, qui est par ailleurs solide à température ambiante (point de fusion 32°C).

Le document US 4 505 997 décrit d'autre part, à titre de composés chimiques nouveaux, des bis-triflylamidures de métaux alcalins, obtenus par échange ionique à partir du tétrabutylammonium correspondant. Ces sels sont des composés solides destinés à être incorporés dans la matrice d'un polymère pour former des électrolytes solides permettant de réaliser des générateurs électrochimiques primaires ou secondaires. En outre ces sels sont décrits comme fortement hydrophiles, propriété résultant, selon les auteurs de l'invention, essentiellement de l'anion bis-triflamidure.

De façon surprenante, on a maintenant découvert qu'un sel formé d'un cation imidazolium substitué et de l'anion bis-triflylamidure est liquide à température ambiante, hydrophobe et possède une viscosité peu élevée favorable à la mobilité ionique, une conductivité assez importante, une grande stabilité thermique (au moins jusqu'à 300°C), une tension de vapeur insignifiante à température ambiante et encore inférieure à 0,1 mbar à 150°C, un large domaine de stabilité électrochimique (au moins de -2V à 2V par rapport au couple iode/triiodure), ensemble de propriétés permettant d'utiliser de tels sels de façon avantageuse comme solvant dans un système électrochimique, telles que les cellules photovoltaïques électrochimiques, ou pour des synthèses nécessitant des solvants polaires aprotiques.

Les composés selon l'invention peuvent être représentés par la formule ci-après : 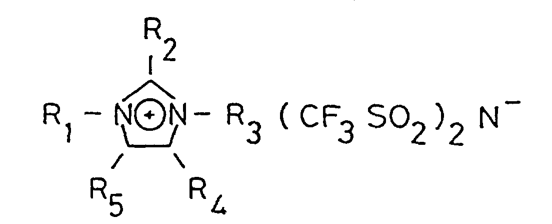 dans laquelle R₁ et R₃ sont identiques ou différents et représentent chacun un radical alkyle linéaire ou ramifié, un radical fluoroalkyle ou un radical alkoxyalkyle de 1 à 8 atomes de carbone; et R₂, R₄ et R₅ sont identiques ou différents et représentent chacun un atome d'hydrogène ou un radical alkyle de 1 à 3 atomes de carbone.

De préférence R₁ ou R₃ représente un radical méthyle, éthyle, butyle, isobutyle, octyle, méthoxyéthyle ou 2,2,2-trifluoroéthyle et R₂, R₄ ou R₅ représente l'hydrogène ou un radical méthyle ou éthyle.

L'invention concerne également un procédé d'obtention desdits composés par échange ionique en milieu aqueux entre un bis-triflylamidure de métal alcalin, de préférence le lithium, et un halogénure d'imidazolium substitué, de préférence le bromure ou l'iodure, selon le schéma réactionnel ci-après :

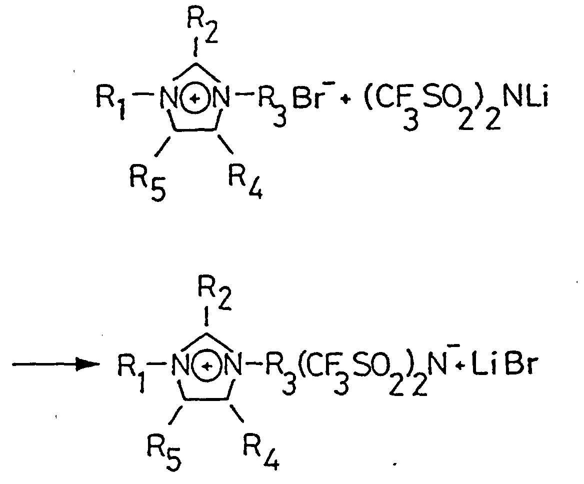

Le bis-triflylamidure de lithium est un composé commercialement disponible, par exemple auprès de la société 3M (St-Paul Minnesota-USA).

Les bis-triflylamidures d'un métal alcalin peuvent également être préparés selon des procédés connus de l'art antérieur, tels que ceux décrits dans le document EP 0 096 629 à partir d'un anhydride perfluoroalcanesulfonique, ou par J. R. Foropoulos et D. D. Desmarteau (Inorg. Chem. 23, 3 720-3723 (1984)) à partir d'un fluorure d'acide perfluoroalcanesulfonique.

L'halogénure d'imidazolium substitué est obtenu selon un procédé connu par action, au sein d'un solvant organique sous agitation, d'un halogènure d'alkyle R₃X sur un imidazole convenablement substitué par R₁, R₂, R₄ et R₅.

Les exemples ci-après donnent à titre illustratif et non limitatif la préparation d'un certain nombre de composés selon l'invention, ainsi que leurs propriétés physico-chimiques et électrochimiques les rendant aptes à être avantageusement utilisés comme solvant électrolytique.

### Exemple 1

### - Bis-triflylamidure de méthyl-1-butyl-3-imidazolium

Dans un réacteur on a dissous 0,1 mole (8,2 g) de méthyl-1 imidazole dans 200 ml de trichloréthane, puis on a ajouté goutte à goutte à température ambiante sous forte agitation une solution de 0,12 mole (16,44 g) de bromure de butyle dans 100 ml de trichloréthane. On a ensuite chauffé à reflux pendant 2 heures, puis laissé le mélange décanter. Le bromure de méthyl-1-butyl-3-imidazolium se sépare sous forme d'un liquide. Le bromure est ensuite lavé deux fois par 100 ml de trichloréthane 50°C, puis séché une heure au Rotavapor à 150°C sous pression réduite de 0,1 mbar pour donner 15,3 g (rendement 70 %) de bromure de méthyl-1-butyl-3-imidazolium.

On a ensuite dissous 50 mmole (11 g) du bromure obtenu à l'étape précédente dans 50 ml d'eau, puis on a ajouté en une seule fois une solution de 50 mmole (14,35 g) de bis-triflylamidure de lithium dans 50 ml d'eau. La solution se trouble immédiatement et après décantation se sépare en deux phases. Après élimination de la phase aqueuse surnageante, on a lavé deux fois le sel liquide avec 50 ml d'eau, puis séché une heure à 150°C sous pression réduite de 0,1 mbar. On a ainsi obtenu 18,9 g de produit du titre (rendement 90 %) se présentant sous forme d'un liquide incolore non miscible à l'eau, ayant pour indice de réfraction n_{D} = 1,4271. Le point de congélation de ce liquide est inférieur à -25°C. Le caractère hydrophobe du produit obtenu est confirmé par une titration selon Karl Fisher du produit préalablement saturé en eau montrant une teneur de seulement 1,4 % en masse.

Une mesure de la densité du composé du titre effectuée à 20°C donne pour valeur d = 1,429 g · cm⁻³, et une mesure de viscosité cinématique, effectuée à 20°C avec un microviscosimètre à bille Haake permet de déterminer la valeur du coefficient de viscosité η = 52,4 cP.
A titre de comparaison le composé homologue connu de l'art antérieur, triflate de méthyl-1-butyl-3-imidazolium est un composé ayant un point de fusion de 16°C, soluble dans l'eau, et de viscosité η = 90 cP.

Le composé du titre s'est également révélé posséder une grande stabilité thermique jusqu'à 300°C et une très faible tension de vapeur (moins de 0,1 mbar à 150°C).

La conductivité du sel liquide mesurée à 20°C avec un potentiostat analyseur de fréquence Autolab a pour valeur σ = 3,88 mS · cm⁻¹, c'est-à-dire une valeur du même ordre de grandeur que l'homologue triflate (σ = 3,68 mS · cm⁻¹).

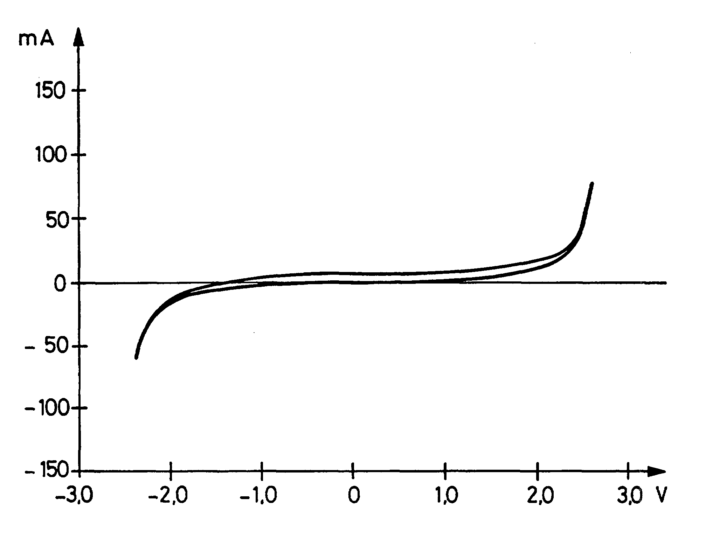

Le cyclovoltammogramme représenté à la figure 1, à effectué à 20°C, avec une électrode de travail en platine d'une surface de 0,78 mm2 et une électrode de référence de ce même métal au contact du couple iode/triiodure dans le même sel liquide, avec un balayage de 50 mVₛ-¹, montre que le composé du titre possède une grande stabilité électrochimique dans une fenêtre comprise entre -2,0 V et +2,2 V.

### Exemple 2

### -Bis-triflylamidure de méthyl-1-éthyl-3 imidazolium

Selon un procédé en deux étapes, comparable à celui de l'exemple 1, mais en remplaçant dans la première étape 0,12 mole de bromure de butyle par 0,25 mole (27,75g) de bromure d'éthyle, on a obtenu 54 g (rendement 78 %) de produit du titre sous forme d'un liquide incolore ayant les caractéristiques physico-chimiques ci-après :

| | |
|---|---|
| Point de congélation | < -25°C; |
| Teneur en eau (Karl Fisher) : | 1,4 % |
| d²⁰ = 1,52 g · cm⁻³ | η = 34,3 cP |
| n_{D} = 1,4231 | σ = 8,84 mS · cm⁻¹ |

Un cyclovoltammogramme réalisé dans les mêmes conditions qu'à l'exemple 1 montre que le composé du titre présente une fenêtre électrochimique comprise entre -2,0 V et +2,2 V.

### Exemple 3

### -Bis-triflylamidure d'éthyl-1-butyl-3-imidazolium

En procédant comme indiqué dans l'exemple 1, mais en partant de 0,1 mole (9,6 g) d'éthyl-1-imidazole on a obtenu 13 g (rendement 60 %) de composé du titre sous forme d'un liquide incolore ayant les caractéristiques physico-chimiques ci-après :

| | |
|---|---|
| Point de congélation | <-25°C |
| Teneur en eau (Karl Fisher) : | 1,3 % |
| d²⁰ = 1,404 g · cm⁻³ | η = 47,6cP |
| η_{D} = 1,4285 | σ = 4,12mS·cm⁻¹ |

De la même façon qu'aux exemples 1 à 3, mais en partant d'imidazoles différemment substitués, on a également préparé les triflylamidures des cations suivants :
méthyl-1-méthyl-3-imidazolium; méthyl-1-méthoxyéthyl-3-imidazolium; méthyl-1-2,2,2-trifluoroéthyl-3-imidazolium; éthyl-1-éthyl-3-imidazolium; méthyl-1-méthyl-2-éthyl-3-imidazolium; éthyl-1-méthyl-2-éthyl-3-imidazolium; méthyl-1-éthyl-3-méthyl-5-imidazolium; éthyl-1-éthyl-3-méthyl-5-imidazolium; éthyl-1-isobutyl-3-imidazolium; méthyl-1-octyl-3-imidazolium.

### Exemple 4

### -Cellule photovoltaïque électrochimique ayant un sel liquide hydrophobe comme solvant électrolytique

On a réalisé une cellule photovoltaïque électrochimique du type de celle décrite dans la demande de brevet internationale WO94/04497, constituée par deux ensembles à électrodes distant de 20 µm, l'une des électrodes étant revêtue d'une couche nanoparticulaire de TiO₂ de 0,3 µm d'épaisseur sur laquelle est absorbé à titre de sensibilisateur le cis-dithiocyanato-bis (2,2'-bipyridyl-4,4'-dicarboxylate ruthénium II) en une quantité telle que l'absorbsion de la lumière visible soit seulement de 5 % vers 520 nm. L'espace libre entre les électrodes a été rempli avec un électrolyte composé du sel liquide de l'exemple 1 comme solvant, de 10 % en poids d'iodure de méthylhexyl imidazolium et de 10 mmole d'iode.

Sous une illumination correspondant à 1/100 de l'illumination solaire standard (AM1) on a obtenu une tension en circuit ouvert de 530 mV et un courant de court-circuit de 27 µA . cm⁻².

En répétant la même expérience avec le sel liquide de l'exemple 2 comme solvant, 10 % en poids d'iodure de méthylhexyl imidazolium et 5 mmole d'iode, on a obtenu une tension en circuit ouvert de 550 mV et un courant de court-circuit de 25 µA . cm⁻².

On a également observé que le caractère hydrophobe du sel liquide utilisé comme solvant électrolytique permettait de réduire la désorption du sensibilisateur de la couche de TiO₂ nanoparticulaire. Contrairement à bien des solvants polaires aprotiques, un sel liquide selon l'invention est inerte envers le triiodure.

Cet exemple est donné à titre illustratif et l'homme du métier est en mesure d'utiliser les sels liquides hydrophobes selon l'invention dans d'autres applications dans le domaine électrochimique sans sortir du cadre de l'invention.

## Revendications

1. Sels liquides hydrophobes de formule générale 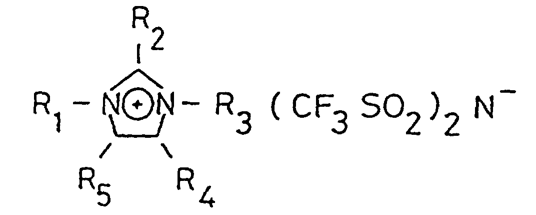 dans laquelle R₁ et R₃ sont identiques ou différents et représentent chacun un radical alkyle linéaire ou ramifié, un radical fluoroalkyle ou un radical alkoxyalkyle de 1 à 8 atomes de carbone; et R₂, R₄ et R₅ sont identiques ou différents et représentent chacun un atome d'hydrogène ou un radical alkyle de 1 à 3 atomes de carbone.

2. Sels liquides hydrophobes selon la revendication 1, **caractérisé en ce que** R₁ ou R₃ représente un radical méthyle, éthyle, butyle, isobutyle, octyle, méthoxyéthyle ou 2,2,2-trifluoroéthyle et R₂, R₄ ou R₅ représente l'hydrogène ou un radical méthyle ou éthyle.

3. Sel liquide hydrophobe selon la revendication 1, **caractérisé en ce qu'**il correspond au bis-triflylamidure de méthyl-1-butyl-3-imidazolium.

4. Sel liquide hydrophobe selon la revendication 1, **caractérisé en ce qu'**il correspond au bis-triflylamidure de méthyl-1-éthyl-3-imidazolium.

5. Sel liquide hydrophobe selon la revendication 1, **caractérisé en ce qu'**il correspond au bis-triflylamidure d'éthyl-1-butyl-3-imidazolium.

6. Procédé de préparation d'un sel liquide hydrophobe selon la revendication 1, **caractérisé en ce qu'**on fait réagir en milieu aqueux un halogénure d'imidazolium substitué par R₁, R₂, R₃, R₄ et R₅ sur un bis-triflylamidure alcalin, et qu'on sépare le produit obtenu par décantation.

7. Procédé de préparation selon la revendication 6 **caractérisé en ce que** l'halogénure est un bromure ou un iodure et le métal alcalin est le lithium.

8. Composition électrolytique contenant à titre de solvant polaire aprotique un sel liquide hydrophobe de formule générale. 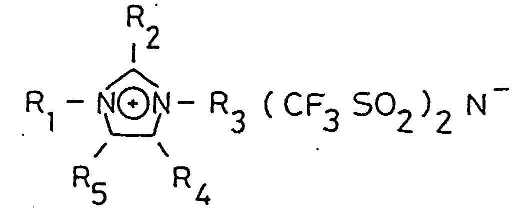 dans laquelle R₁ représente un radical alkyle de 1 à 5 atomes de carbone, R₃ représente un radical alkyle linéaire ou ramifié, un radical fluoroalkyl ou un radical alkoxyalkyle de 1 à 8 atomes de carbone; et R₂, R₄ et R₅ représentent séparément un atome d'hydrogène ou un radical alkyle de 1 à 3 atomes de carbone.

9. Cellule photovoltaïque électrochimique contenant une composition électrolytique selon la revendication 8 associée au couple iode/triiodure.

## Patentansprüche

1. Hydrophobe flüssige Salze mit der allgemeinen Formel 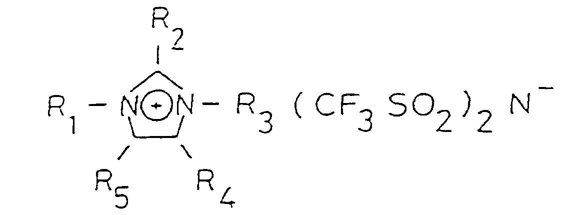 in der R₁ und R₃ gleich oder unterschiedlich sind und jeweils für einen linearen oder verzweigten Alkylrest, einen Fluoralkylrest oder einen Alkoxyalkylrest mit 1 bis 8 Kohlenstoffatomen stehen; und R₂, R₄ und R₅ gleich oder unterschiedlich sind und jeweils ein Wasserstoffatom oder ein Alkylrest mit 1 bis 3 Kohlenstoffatomen sind.

2. Hydrophobe flüssige Salze nach Anspruch 1, **dadurch gekennzeichnet, dass** R₁ oder R₃ ein Methyl, Ethyl, Butyl, Isobutyl, Octyl, Methoxyethyl oder 2,2,2-Trifluorethyl-Rest darstellen, und dass R₂, R₄ oder R₅ Wasserstoff oder ein Methyl- oder ein Ethylrest sind.

3. Hydrophobes flüssiges Salz nach Anspruch 1, **dadurch gekennzeichnet, dass** es das bis-Triflylilamid von 1-Methyl-3-butyl-imidazolinium ist.

4. Hydrophobes flüssiges Salz nach Anspruch 1, **dadurch gekennzeichnet, dass** es das bis-Triflylilamid von 1-Methyl-3-ethyl-imidazolinium ist.

5. Hydrophobes flüssiges Salz nach Anspruch 1, **dadurch gekennzeichnet, dass** es das bis-Triflylilamid von 1-Ethyl-3-butyl-imidazolinium ist.

6. Verfahren zum Herstellen eines hydrophoben flüssigen Salzes nach Anspruch 1, **dadurch gekennzeichnet, dass** man in wässrigem Medium ein durch R₁, R₂, R₃, R₄ und R₅ substituiertes Imidazolinium-Halogenid mit einem Alkali-bis-triflylilamid reagieren lässt, und man das erhaltene Produkt durch Dekantieren abtrennt.

7. Verfahren zur Herstellung nach Anspruch 6, **dadurch gekennzeichnet, dass** das Halogenid Brom oder Iod ist und dass das Alkalimetall Lithium ist.

8. Elektrolytische Zusammensetzung, das als aprotisches polares Lösemittel ein hydrophobes flüssiges Salz der allgemeinen Formel 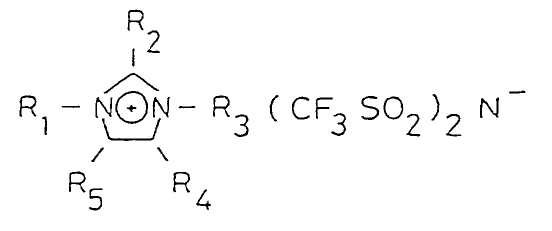 aufweist, in der R₁ für einen Alkylrest mit 1 bis 5 Kohlenstoffatomen, R₃ für einen linearen oder verzweigten Alkylrest, ein Fluoralkylrest oder ein Alkoxyalkylrest mit 1 bis 8 Kohlenstoffatomen steht; und R₂, R₄ und R₅ unabhängig voneinander ein Wasserstoffatom oder ein Alkylrest mit 1 bis 3 Kohlenstoffatomen bedeuten.

9. Elektrochemische photovoltaische Zelle, enthaltend eine elektrolytische Zusammensetzung nach Anspruch 1 in Zusammenhang mit dem Paar Iod/Triiodid.

## Claims

1. Hydrophobic liquid salts of general formula 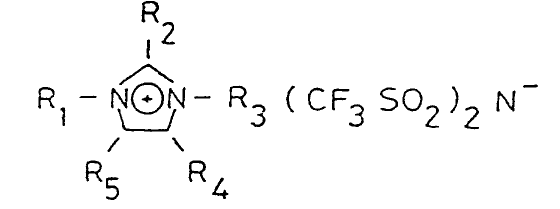 in which R₁ and R₃ are identical or different and each represent a linear or branched alkyl radical, a fluoroalkyl radical or an alkoxyalkyl radical containing from 1 to 8 carbon atoms; and R₂, R₄ and R₅ are identical or different and each represent a hydrogen atom or an alkyl radical containing from 1 to 3 carbon atoms.

2. Hydrophobic liquid salts according to Claim 1, **characterized in that** R₁ or R₃ represents a methyl, ethyl, butyl, isobutyl, octyl, methoxyethyl or 2,2,2-trifluoroethyl radical and R₂, R₄ or R₅ represents hydrogen or a methyl or ethyl radical.

3. Hydrophobic liquid salt according to Claim 1, **characterized in that** it corresponds to methyl-1-butyl-3-imidazolium bistriflylimide.

4. Hydrophobic liquid salt according to Claim 1, **characterized in that** it corresponds to methyl-1-ethyl-3-imidazolium bistriflylimide.

5. Hydrophobic liquid salt according to Claim 1, **characterized in that** it corresponds to ethyl-1-butyl-3-imidazolium bistriflylimide.

6. Process for preparing a hydrophobic liquid salt according to Claim 1, **characterized in that** an imidazolium halide substituted with R₁, R₂, R₃, R₄ and R₅ is reacted with an alkali metal bistriflylimide, in aqueous medium, and the product obtained is separated by settling out.

7. Preparation process according to Claim 6, **characterized in that** the halide is a bromide or an iodide and the alkali metal is lithium.

8. Electrolyte composition containing, as aprotic polar solvent, a hydrophobic liquid salt of general formula 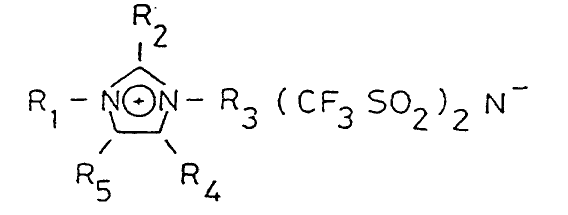 in which R₁ represents an alkyl radical containing from 1 to 5 carbon atoms, R₃ represents a linear or branched alkyl radical, a fluoroalkyl radical or an alkoxyalkyl radical containing from 1 to 8 carbon atoms; and R₂, R₄ and R₅ represent, separately, a hydrogen atom or an alkyl radical containing from 1 to 3 carbon atoms.

9. Electrochemical photovoltaic cell containing an electrolyte composition according to Claim 8 combined with the iodine/triiodide couple.
